# EUROPEAN PATENT SPECIFICATION

(11) **EP 3 114 485 B1**
(45) Date of publication and mention of the grant of the patent: **18.11.2020**
(21) Application number: 15709334.5
(22) Date of filing: 06.03.2015
(51) Int. Cl.: G01N 33/68, C07K 16/18

(54) **NEPHROPATHY BIOMARKER**
NEPHROPATHIEBIOMARKER
BIOMARQUEUR DE LA NÉPHROPATHIE

(30) Priority: 07.03.2014 GB 201404061
(43) Date of publication of application: 11.01.2017
(73) Proprietor: Epsom And St Helier University Hospitals NHS Trust, Surrey SM5 1AA (GB)
(72) Inventor: DOCKRELL, Mark, Carshalton Surrey SM5 1AA (GB); SHAH, Nileshkumar, Carshalton Surrey SM5 1AA (GB)
(74) Representative: CSY London
(86) International application number: PCT/GB2015/050669
(87) International publication number: WO 2015/132612

(56) References cited:
- WO-A1-2009/055937
- US-A1- 2011 177 613
- US-A1- 2013 338 021
- CHO EUN AH ET AL: "Differential expression and function of cadherin-6 during renal epithelium development", DEVELOPMENT (CAMBRIDGE), vol. 125, no. 5, March 1998 (1998-03), pages 803-812, XP002738137, ISSN: 0950-1991
- Anonymous: "Helier Scientific Ltd announced as one of the 14 winners of the NIHR Devices for Dignity HTC SBRI Kidney Care Competition. Kidney care receives innovation boost", Helier Scientific Research News , 13 May 2014 (2014-05-13), XP002738148, Retrieved from the Internet: URL:http://www.helierscientific.co.uk/news .html [retrieved on 2015-04-07]

## Description

The invention relates to a biomarker useful for the diagnosis of kidney disease and for determining patients whose disease severity level is likely to progress. Accordingly, the invention relates to a method for determining the likelihood of kidney disease progression, particularly of chronic kidney disease in a diabetic patient.

Chronic kidney disease (CKD) and, in its extreme form, end stage renal failure are conditions associated with significant morbidity and mortality. The incidence of CKD is significant and, with aging and more diabetes-prone populations, is rising internationally.

CKD is a condition of varying severity which frequently progresses along a 'severity spectrum' over time. Stages 1 and 2 may be considered mild CKD, stage 3 moderate and stages 4 and 5 more severe. Stage 5 may be considered end-stage/established renal failure (ERF).

In addition to the direct effects on kidney function (which might be relatively asymptomatic for mild forms), the CKD is associated with an increased risk of heart disease and stroke.

In the UK, the cost to the National Health Service of renal replacement therapy (RRT), for patients with ERF, is significant. Further, such costs are expected only to increase with the rising rates of ERF; according to UK renal registry data the incidence of ERF requiring RRT increased by approximately 60% between 1991-1992 and 2008.

In addition to cost implications, the quality-of-life impact of dialysis and transplantation renal replacement therapies on patients is significant, and so they are desirable to avoid wherever possible.

Not everyone with CKD will progress to advanced forms of the disease, however. Accordingly, early identification of patients at risk of developing advanced renal damage (e.g. ERF) could be highly advantageous, by, for example, appropriately prompting for intervention to be taken to slow/arrest disease progression.

CKD has a range of causes, including high blood pressure, diabetes, general aging, glomeruli inflammation, polycystic kidney disease, repeated infection, renal artery stenosis and drug/toxin damage. Of these, diabetes is considered one of the most significant, with diabetic nephropathy (diabetes-related kidney disease) accounting, in the West, for around 30% of patients requiring RRT. This impact is most likely only to concomitantly rise with the increasing obesity rates of many countries.

Though there may be distinct underlying CKD causes, however, there are common indicators used to diagnose and assess kidney disease status.

Tubulo-interstitial fibrosis (deposition of excess connective tissue on the kidney parenchyma), for example, contributes to the irreversible deterioration of renal function and is an accurate predictor of long-term prognosis, irrespective of the type of disease or the compartment of origin. It is assessed by renal biopsy, however, a procedure which can incur complications and risks and so is neither desirable nor feasible for all patients.

Another indicator, a decrease in glomerular filtration rate (GFR), is measured by using a radioactive tracer and is considered invasive and not cost-effective. Use of estimated GFR (eGFR) lacks sensitivity and specificity for diagnosis and prognosis of diabetic nephropathy.

The current "gold standard" indicator for (diabetic) CKD, therefore, may be considered to be a person's urinary albumin (or other/general) protein level. For example, microalbuminuria, when the kidneys 'leak' albumin into the urine and which may be related to failure of proximal-tube endocytosis, can be assessed by a non-invasive urine test. (Albuminuria is a sub-type of, and may precede, proteinuria.) Furthermore, to compensate for possible gross protein variation between samples, a person's protein/albumin to creatinine ratio (PCR/ACR) may be determined.

Though an increasing urine albumin/protein level is an indicator of kidney decline, urine albumin/protein *per se* is considered to have limited sensitivity and specificity with respect to CKD progression. For diabetic nephropathy, for example, not all patients with microalbuminuria will progress to ERF and a significant proportion of type-2 diabetic patients do not present with incremental proteinuria or have non-proteinuric renal disease (yet, of these, a substantial proportion progress over time).

In view of matters such as those discussed above, there is an ever-increasing demand for a convenient and reliable biomarker which allows for early identification of subjects with CKD that is likely to progress. A predictive assay could be highly advantageous by allowing for preventative patient care strategies to be pre-emptively adopted.

According to an aspect of the present invention there is provided a method for determining the propensity for a person's kidney disease (CKD) level to progress in severity, the method comprising testing for the presence/level of K-cadherin in the person's urine. In this regard, it has been found that detection (e.g. by Western immunoblot as described below) of urinary K-cadherin indicates a person is likely to progress along the severity spectrum. It is also considered that the relative level of urinary K-cadherin may be related to the rate of progression. Further, as discussed further below, the relative K-cadherin level is pertinent with respect to more sensitive systems (such as an ELISA (enzyme-linked immunosorbent assay) that has proven able to detect its presence in non-progressors' urine, for example. The invention therefore provides for an assay useful for determining whether a person's CKD is likely to progress, and thus whether, for example, closer monitoring/intervention treatment may be advisable.

It will be understood that K-cadherin relates to 'normal' expressed, un-fragmented protein (since, as mentioned below, fragments which react with K-cadherin antibodies have been found in the urine of healthy control subjects). Accordingly, the K-cadherin may be thought of/described as full length (as judged by electrophoresis separation, for example) though, as would be apparent to a skilled person, there could naturally be a level of variation.

In this way, if a person is found to have a greater likelihood of progressing towards ERF then clinicians could plan and implement management and prevention strategies, as appropriate for the patient, in order to try and slow/arrest disease progression. Testing a person's urine is also significantly less intrusive than available methods for assessing kidney health.

K-cadherin, which is also known as cadherin-6 (CDH6), is a member of the cadherin protein family, a family, having more than 100 members, of transmembrane glycoproteins important in the maintenance of cell-cell adhesion, cell phenotype regulation, tissue organisation and development. K-cadherin is a 120kDa type II classical cadherin of 790 amino acids, coded by the CDH6 gene on chromosome 5. As described below, full length K-cadherin (as measured by Western Immunoblotting) was not detected in the urine of healthy control subjects or diabetics without CKD but was detectable initially (and subsequently) in the urine of every patient who exhibited progressive CKD over the study period. Further, none of the diabetic CKD subjects who were negative for urinary K-cadherin at baseline progressed over the follow-up period.

Additionally, as measured by a specifically developed ELISA disclosed below (that allows for greater quantification in comparison to Western blotting), while non-progressors were found to exhibit full-length urinary K-cadherin, the mean levels were approximately half those seen for progressors at corresponding CKD stages.

Accordingly, the method may comprise testing for elevated K-cadherin. The elevated K-cadherin may be with respect to the normal (healthy) population and/or relative to a non-progressive standard model (control). There may be different non-progressive standard levels applicable to different CKD disease stages; stages may include mild disease (CKD stages 1/2) and/or moderate/severe disease (CKD stages 3/4).

Elevation may also optionally be assessed more specifically, e.g. with respect to a control non-progressive standard for particular variable characteristics (used to calculate estimated renal function) common to a patient. Such characteristics may include any one or more of age, ethnicity, gender and traceable IDMS (isotope-dilution mass spectrometry).

In some instances the degree of elevation for indicating likelihood of progression may correspond to at least an approximate doubling of the relevant non-progressive level. Other possibilities include instances where a non-progressive standard may correspond to an undetectable K-cadherin concentration.

Further, it has been shown that normal human K-cadherin expression is (along with N-cadherin/cadherin-2) localised to the proximal renal tubules, and that in diabetic kidney biopsies there is a general reduction in proximal tubule cadherin expression which seemingly correlates with the degree of tubular interstitial fibrosis.

Above characteristics of K-cadherin were, however, not echoed for N-cadherin (cadherin-2). In this regard, full-length N-cadherin protein was present in the urine of all subjects tested (including healthy controls) and did not correlate with CKD, even though it was found (as for K-cadherin) to be downregulated in proximal tubule epithelial cells in kidney biopsies from patients with diabetic CKD. This may relate to its widespread expression, particularly in the lower urinary tract and the prostate.

The person(s) (patients) may be diabetics. Further, the kidney disease may be diabetic chronic kidney disease (diabetic nephropathy). The kidney disease may also be, for example, IgA nephropathy or chronic allograft nephropathy (whether or not the person is diabetic). In this regard, some of the relevant diabetic patients in the study described below may not have had diabetic nephropathy, they may coincidentally have had another form of CKD (though efforts were made to avoid this and it is expected that for most/all cases the CKD cause would have been diabetes). Moreover, K-cadherin loss similar to that seen from proximal tubule epithelia in early diabetic CKD has also been observed for advancing IgA nephropathy and chronic allograft nephropathy (CAN). Furthermore, loss of K-cadherin was observed also in an *in vitro* model of fibrosis using primary human proximal tubule cells.

A person for whom the invention may be used may have more than cause/type of kidney disease.

The person may be a type I or type II diabetic.

Testing for K-cadherin may conveniently involve Western Immunoblotting as a detection system. In this regard, it has been shown that K-cadherin was not detected by Western Immunoblotting in the urine of healthy control subjects or diabetics without CKD but was detectable initially (and subsequently) in the urine of every patient who exhibited progressive diabetic CKD over the study period. Other techniques which may be used/exploited include an antibody system such as an ELISA (enzyme-linked immunosorbent assay), NMR (nuclear magnetic resonance), colorimetric and mass spectrometry.

A point of care system, e.g. one exploiting a membrane-based lateral flow strip (similar to the approach used in pregnancy tests), could be used to facilitate result determination. Such a test system may optionally exploit one or more antibodies as discussed below.

Preferably, the method comprises Western Immunoblotting and/or an ELISA for assessing the presence and/or level of K-cadherin. It will be apparent to a person skilled in the art that for embodiments using Western immunoblotting the sample should first be subject to a process such as gel electrophoresis, e.g. SDS-PAGE.

For embodiments comprising an ELISA, the ELISA system used preferably comprises two sets of antibodies. Preferably, one set is reactive towards the N-terminal end of K-cadherin and the other towards the C-terminal end. In this way, the target protein may be immobilised from one end and detected by antibodies which interact with the other end and, accordingly, the assay effectively tests for the presence of essentially full-length (non-degraded) K-cadherin. In this regard, it was hypothesised that cellular degradation and death might lead to cleavage of K-cadherin and excretion of the extra-cellular domain (with the cytoplasmic domain being endocytosed and recycled/degraded in the proteasome). Surprisingly, however, full-length K-cadherin (as judged on a Western Immunoblot) was found in the urine of diabetic CKD patients, unlike for healthy controls. This implied the release of intact, essentially full length K-cadherin from proximal tubular epithelial cells exposed to fibrotic stimuli. Protein fragments recognised by K-cadherin antibodies have also been found in healthy control urine, indicating metabolism/degradation and excretion of K-cadherin may be a normal pathway.

Another option for an antibody-based detection system such as an ELISA may be to use an antibody specific to the relevant K-cadherin cleavage point, e.g. as the capture antibody. By recognising and binding only protein un-broken at the relevant point, the presence of full-length K-cadherin may specifically be detected.

Conveniently the urine sample may be concentrated before the presence (and level) of K-cadherin is assessed. Preferably, a sample is concentrated six-fold. In this regard, it has been found that concentrating a sample can facilitate subsequent testing (e.g. by reducing the sample volume to be applied to a gel). Whether or not, and to what extent, it is desirable to concentrate a sample may vary and depend on the testing method and the starting urine concentration. For example, as discussed below, urinary K-cadherin has been detected by ELISA in samples that were initially concentrated but then re-diluted (to the corresponding extent) to decrease urine matrix interference.

Optionally, the method further comprises testing for albumin and/or other protein in the person's urine. In this regard, (micro)albuminuria, in the early stage, and subsequently proteinuria, are the current favoured options as markers for the diagnosis and progression of diabetic nephropathy (and may also be applied to other forms of kidney disease). Furthermore, it was found that for diabetics with CKD≥3 (eGFR <60 ml/min), all the subjects that progressed (whose CKD progressed) over a three year period had both detectable urinary K-cadherin and albuminuria at baseline.

According to another aspect of the present invention there is provided use of urinary K-cadherin as a marker for determining the propensity for a person's kidney disease level to progress in severity. Furthermore, it will be apparent to a person skilled in the art that optional features discussed with respect to the first aspect may equally apply with respect to the use herein disclosed.

According to another aspect of the present invention there is provided a kit for testing for the presence/level of K-cadherin in a person's urine. Also, according to another aspect of the present invention there is provided a point-of-care test for testing for the presence/level of K-cadherin in a person's urine.

The point-of-care test and/or the kit may comprise a K-cadherin cleavage-point specific antibody. The point-of-care test and/or the kit may comprise two sets of K-cadherin antibodies. Preferably, one of the antibody sets is reactive towards the N-terminal end of K-cadherin and the other is reactive towards the C-terminal end. The kit may conveniently be for a Western blotting assay (e.g. one involving immunoprecipitation) or an ELISA assay. Conveniently the antibodies are commercially available, e.g. Abcam Anti-K-cadherin antibody ab64917, Thermo Scientific Anti K-cadherin monoclonal antibody MA1-06305, Cadherin K antibody (2B6) from ThermoScientific and Anti-K Cadherin antibody (ab68342) from Abcam.

An example embodiment(s) of the invention will now be described with reference to the accompanying Drawings, of which:

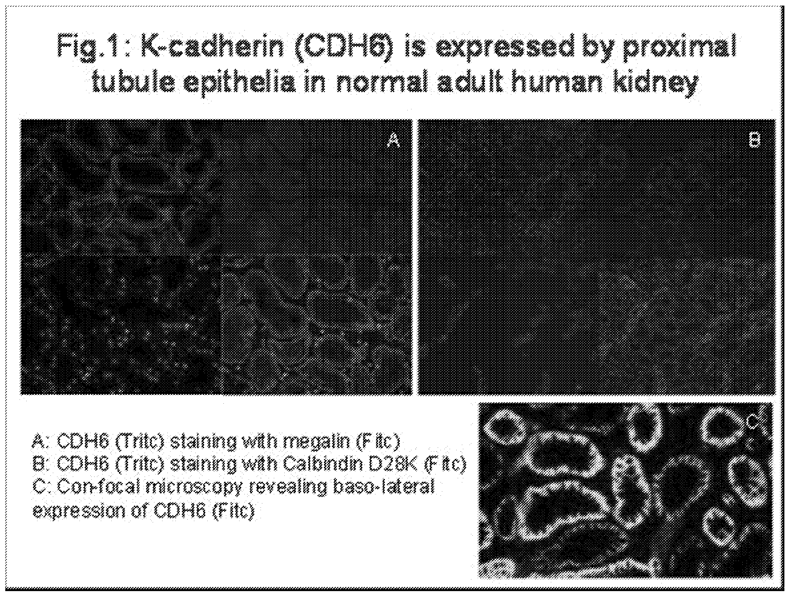

Figure 1 shows K-cadherin (CDH6) immunofluorescence staining of normal adult kidney biopsy with markers of proximal tubule (megalin) and distal tubule (Calbindin D28K): (A) Co-localisation of K-cadherin with megalin; (B) Absence of co-localisation of K-cadherin and calbindin D28K. This suggests K-cadherin is expressed by proximal tubule epithelia in normal adult kidney at the baso-lateral surface. Figure 1(C) shows a confocal microscopy image revealing baso-lateral expression of CDH6 (Fitc). In part A; the Trite-stained image is at the top left when the Figure is orientated upright, the Fitc-stained image is at the top right, the bottom left image shows nucleus staining and the bottom right image is a composite of the others. In part B; the Trite-stained image is at the top right when the Figure is orientated upright, the Fitc-stained image is at the bottom left, the top left image shows nucleus staining and the bottom right image is a composite of the others.

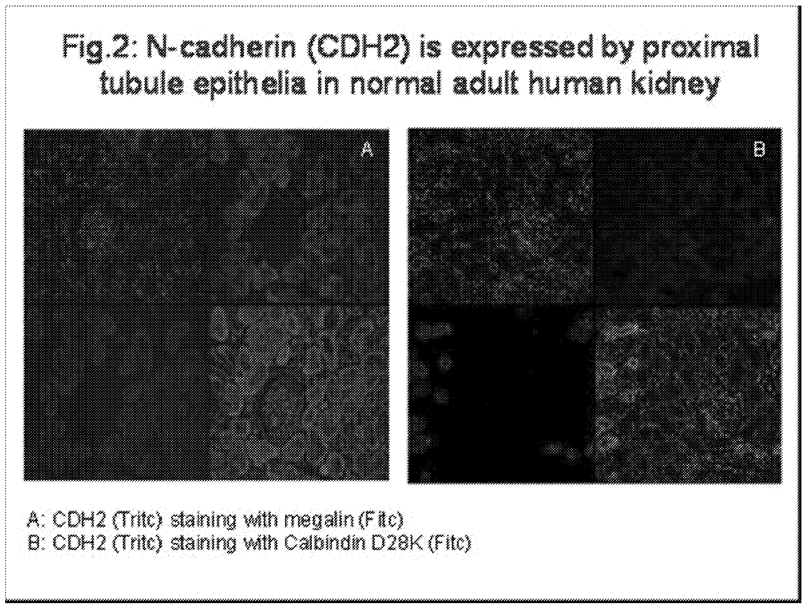

Figure 2 shows N-cadherin (CDH2) immunofluorescence staining of normal adult kidney biopsy with markers of proximal tubule (megalin) and distal tubule (Calbindin D28K): (A) Co-localisation of N-cadherin with megalin; (B) Absence of co-localisation of N-cadherin and calbindin D28K. This suggests N-cadherin is expressed by proximal tubule epithelia in normal adult kidney.

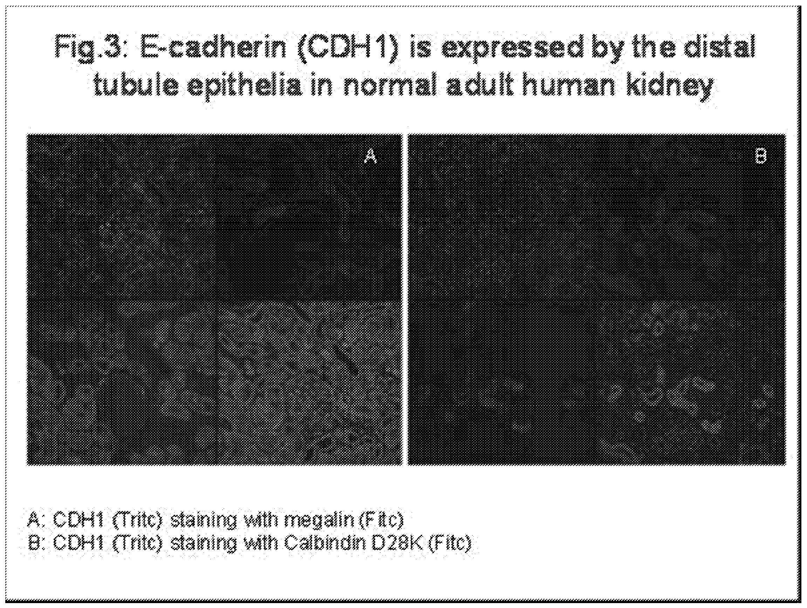

Figure 3 shows E-cadherin (CDH1) immunofluorescence staining of normal adult kidney biopsy with markers of proximal tubule (megalin) and distal tubule (Calbindin D28K): (A) Co-localisation of E-cadherin with calbindin D28K; (B) Absence of co-localisation of E-cadherin and megalin. This suggests E-cadherin is expressed by the distal tubule epithelia in normal adult kidney.

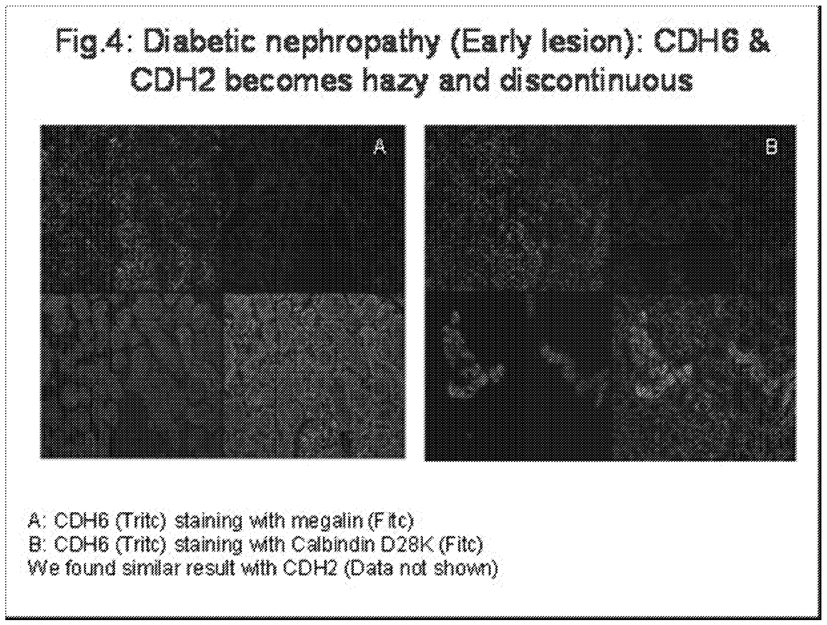

Figure 4 shows K-cadherin (CDH6) & N-cadherin (CDH2) immunofluorescence staining in human kidney biopsy of diabetic nephropathy (Early lesion) with markers of proximal tubule (megalin) and distal tubule (Calbindin D28K) respectively. K-cadherin staining (megalin- PT) in human proximal tubule of diabetic nephropathy (early lesion) became hazy and discontinuous (A) as compared to proximal tubule of healthy adult human kidney (B). Similar findings were observed for N-cadherin in early lesions of proximal tubule of diabetic nephropathy as compared to healthy adult human kidney (data not shown).

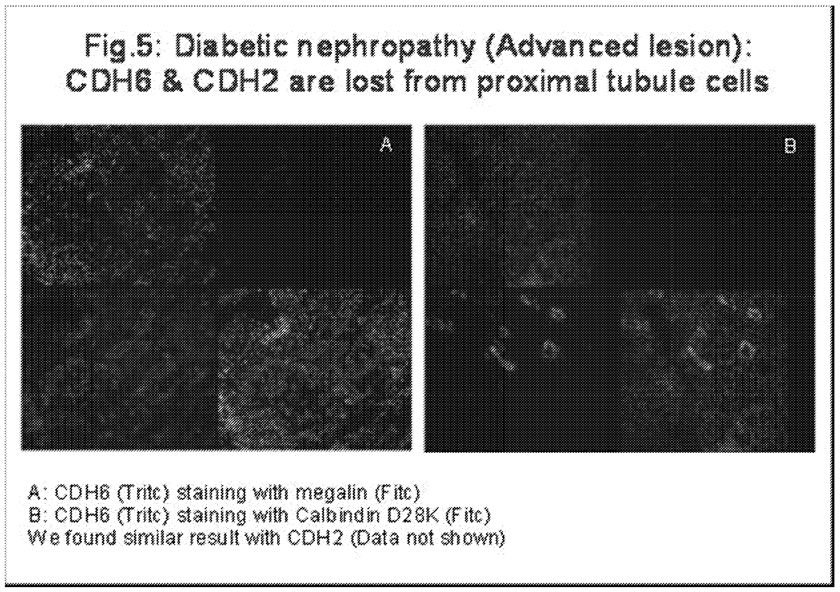

Figure 5 shows K-cadherin (CDH6) immunofluorescence staining in human kidney biopsy of diabetic nephropathy (Advanced lesion) with markers of proximal tubule (megalin) and distal tubule (Calbindin D28K) respectively. K-cadherin (megalin-PT) (A) and K-cadherin (calbindin D28K) (B) staining in human proximal tubule of diabetic nephropathy with advanced lesion was completely lost from the proximal tubule. Similar findings were observed for N-cadherin in advanced lesions of proximal tubule of diabetic nephropathy (data not shown).
   For each of Figures 2 to 5, in both parts A and B, the Tritc-stained image is at the top right when the Figure is orientated upright, the Fitc-stained image is at the bottom left, the top left image shows nucleus staining and the bottom right image is a composite of the others.

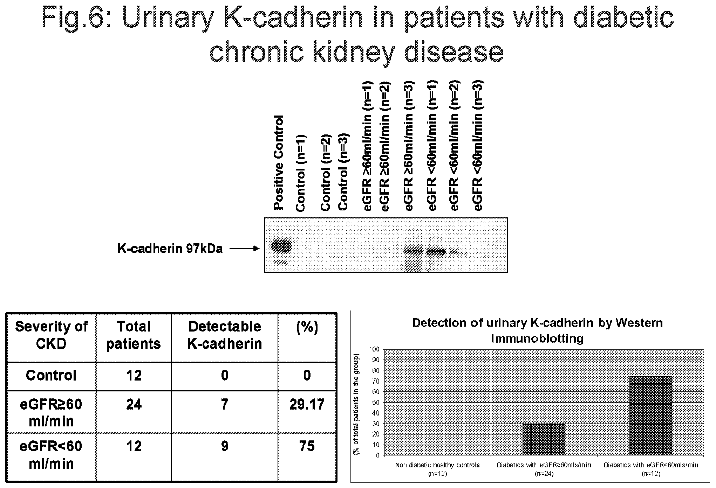

Figure 6 shows a Western Immuno-blot of a subset of urine samples showing urinary full length K-cadherin in patients with diabetic CKD. The table and graph show the number and percentage of patients in each group with detectable K-cadherin in the urine on enrolment in the study. Around 30% of diabetic patients with an eGFR≥60mls/min (Early CKD) had detectable urinary K-cadherin as compared to almost 70% patients with an eGFR<60 mls/min (Advanced CKD) as compared to none in healthy controls.

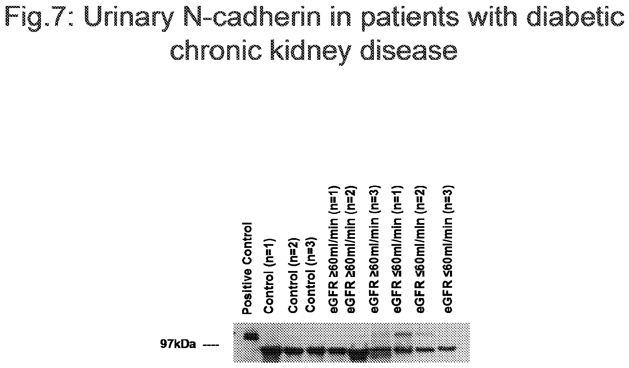

Figure 7 shows a Western Immuno-blot for urinary N-cadherin. In contrast to urinary full length K-cadherin, full length N-cadherin was almost universally present in diabetic patients with an eGFR≥60mls/min (Early CKD), with an eGFR<60 mls/min (Advanced CKD) as well as in urine of healthy controls.

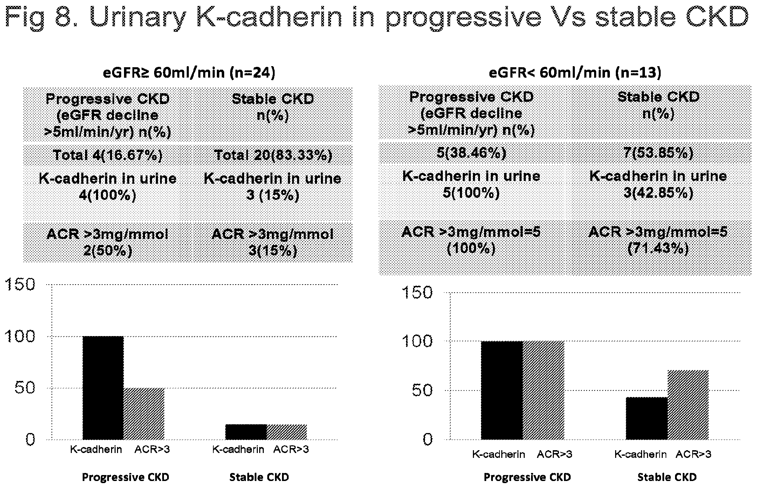

Figure 8 shows urinary K-cadherin and albumin in patients with progressive CKD as compared to stable CKD in early CKD (eGFR≥60mls/min) versus advanced CKD (eGFR<60mls/min). Presence of both K-cadherin and albumin in urine predict progression of CKD over 3 years follow up.

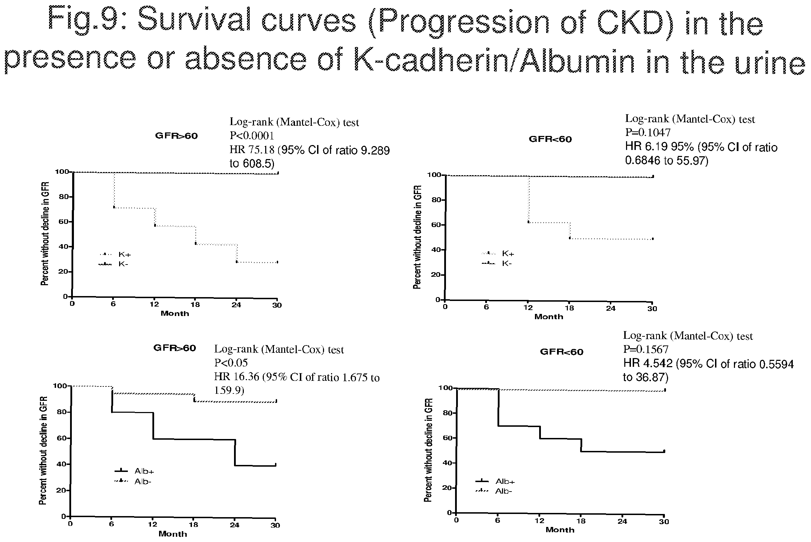

Figure 9 shows the presence or absence of K-cadherin or albumin in the urine of patients with an eGFR≥ 60mls/min (early CKD) or eGFR<60mls/min (advanced CKD) plotted using a Kaplan-Meier survival curve, where an event was defined as a progression of CKD. For each of the graphs the 'positive' (K-cad/albumin) line was the lower line. Early CKD patients with K-cadherin in the urine had a statistically significant risk of progression of CKD over the follow up period (p<0.0001, HR-75.18 (95% CI of ratio 9.289 to 608.5)), similar to early CKD patients with albumin in the urine over this follow up period (p<0.05, HR 16.36 (95% CI of ratio 1.675 to 159.9)). In contrast to the early CKD group, neither advanced CKD patients with K-cadherin nor advanced CKD patients with albumin in the urine had a statistically significant risk of progression of CKD over this follow up period [(p=0.1047, HR 6.19 95% (95% CI of ratio 0.6846 to 55.97)) and (p=0.1567, HR 4.542 (95% CI of ratio 0.5594 to 36.87)) respectively]. This may be due to small the sample size of this group.

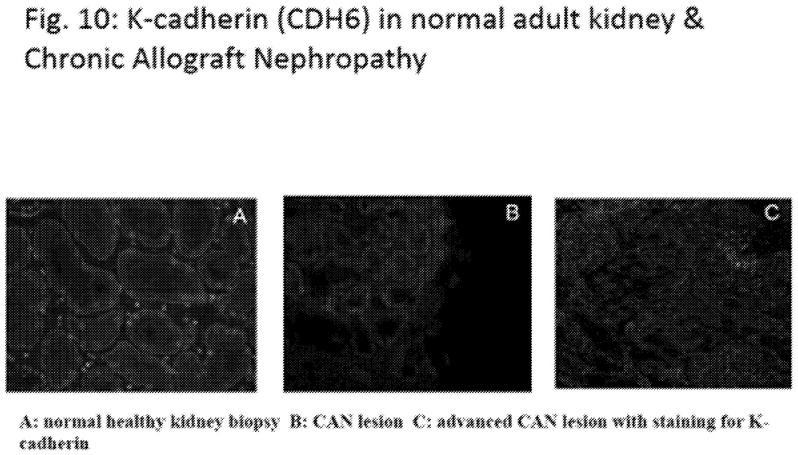

Figure 10 shows data demonstrating the loss of K-cadherin in CAN-affected kidney.

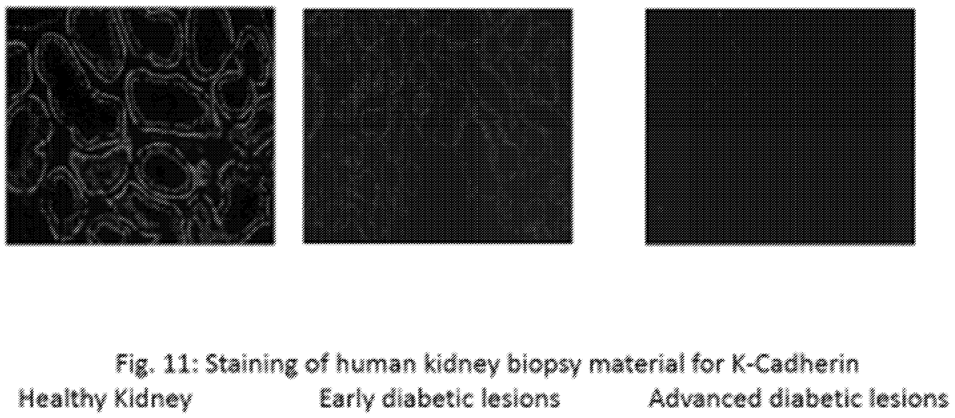

Figure 11 shows a further representation of data demonstrating the progressive loss of K-cadherin in diabetic lesions by comparing expression in healthy kidneys and kidney tissue at various stages of disease.

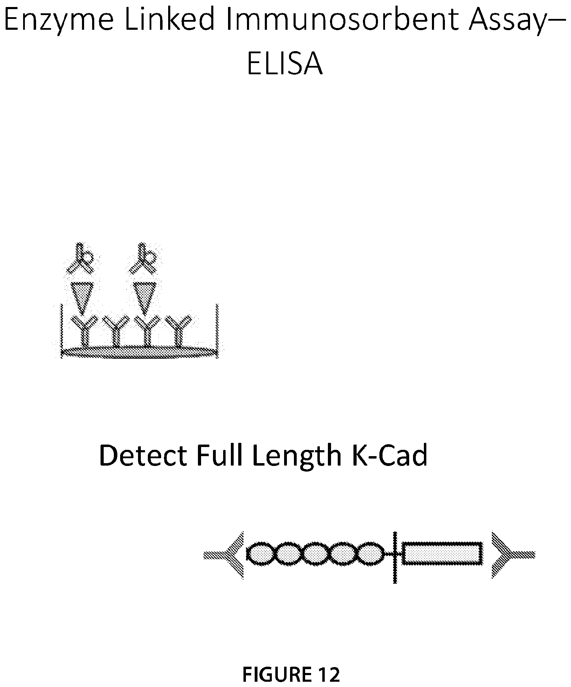

Figure 12 indicates schematically how an example ELISA assay according to the invention may operate.

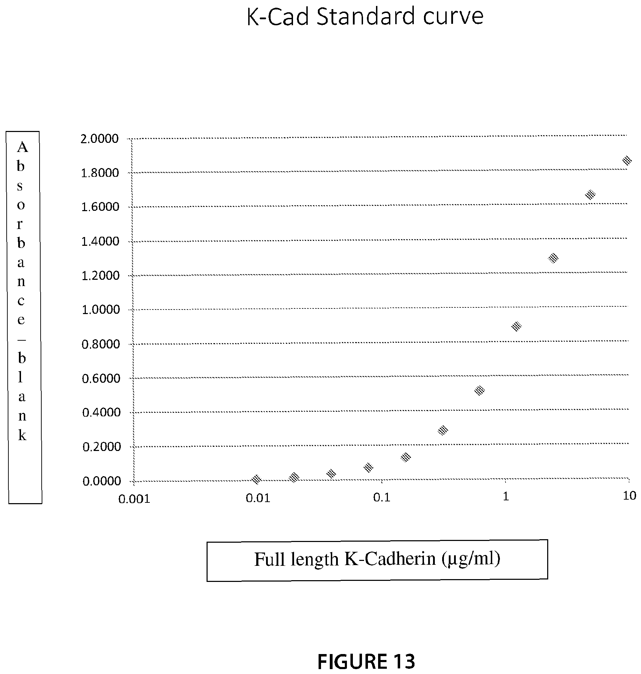

Figure 13 shows a standard curve achieved for an example ELISA assay.

### Example 1: Immuno-histochemistry of archived human kidney biopsy.

Paraffin blocks of archived human kidney biopsy tissue samples were selected based on the tissue diagnosis report. Cadherin expression was investigated in the context of tubulo-interstitial damage and compared to biopsies reported as histologically normal with no evidence of tubulo-interstitial fibrosis (n=5). This work was carried in the department of Pathology, University Medical Centre, Utrecht, with local ethical approval. Briefly, 3µm thick serial sections were made from each paraffin block using a sliding microtome and the sections mounted on super frost plus slides (Thermo Scientific). The sections were fixed on a slide warmer. Deparaffinization and rehydration of the sections were performed. Blocking to remove endogenous tissue peroxidase was performed by oxidizing and using freshly prepared peroxidase block buffer (3% H₂O₂ in PBS) . Antigen retrieval was performed using Tris/EDTA (pH9.0) at boiling temperature for 20 min. The sections were incubated over night at 4°C with monoclonal antibodies against extracellular domain of K-cadherin (diluted 1:40 in Phosphate Buffered Saline, Fischer Thermo-scientific, UK, Cadherin K Antibody 2B6), N-cadherin (1:400, Sigma-Aldrich, UK) and E-cadherin (1:40, Novocastra Laboratories, UK) along with polyclonal antibodies against markers of proximal tubule cells(megalin (1:50, Santa Cruz Biotechnologies, USA)) and distal tubule cells (Calbindin D28K (1:20, Santa Cruz Biotechnologies, USA)). For calbindin D28K, donkey anti-goat AlexaFluor 488 (FITC) (1:200, Invitrogen, USA) was used as secondary antibody. All other primary antibodies were detected using poly-HRP-anti-rabbit/mouse/goat IgG Power Vision (Immunologic, Duiven, The Netherlands), according to manufacturer's instructions, for an hour at room temperature and amplified using the TSA (Tyramide signal amplification) tetra methyl rhodamine system (Tritc) or TSA fluorescein system (Fitc) (Perkin Elmer Life Sciences; Waltham, MA) diluted 1:50 with the amplification buffer. All sections were analyzed using a digital high resolution epi-fluorescence microscope (Leica Microsystems; Rijswijk, The Netherlands).

### Results

Expression profile of cadherins in human kidney.

Immuno-staining of normal human biopsy tissue revealed expression of K and N-cadherin in the proximal tubule; as determined by co-localisation with the large endocytosis receptor megalin (Figures 1 and 2). E-cadherin was expressed in the distal tubule; determined by co-localisation with calbindin D28K (a marker of the distal tubule) (Figure 3). Confocal laser scanning microscopy indicated baso-lateral staining for K- and N-cadherins using antibodies targeting the extracellular domain (ECD) (Figure 1C). This highlights the species specificity of cadherin expression as E-cadherin is expressed in the proximal tubule of rodents (data not shown) but not in humans.

In early diabetic nephropathy lesions, expression of K- and N-cadherin on the basal side was hazy and discontinuous (Figure 4). In advanced lesions with marked tubulo-interstitial fibrosis, K- and N-cadherin expression was lost (Figure 5). Figure 11 brings such data together and shows the progressive nature of K-cadherin loss. E-cadherin expression was also lost but only in sections exhibiting a phenotype characteristic of later stages of tubulo-interstitial fibrosis (data not shown).

Based on unpublished *in vitro* findings (from previous work and work by others) that N-cadherin is up-regulated by the powerful fibrotic growth factor TGFβ1, it was also expected that N-cadherin would be up regulated in patients with advanced tubulo-interstitial fibrosis. Instead, N-cadherin down-regulation in kidney biopsies from patients with diabetic CKD was found.
This study demonstrates for the first time that expression of K- and N-cadherin are altered in the proximal tubule epithelia in early diabetic CKD and that the loss is progressive with advancing tubulo-interstitial fibrosis. Further data suggests that this phenomenon is true irrespective of the etiology of the fibrotic process, e.g. similar K-cadherin loss was observed with advancing IgA nephropathy and chronic allograft nephropathy (CAN) (Figure 10 shows results for CAN). Moreover, loss of K-cadherin was observed also in an *in vitro* model of fibrosis using primary human proximal tubule cells.

This Example also demonstrates that the predominant cadherin in human proximal tubules is K-cadherin and not E-cadherin.

### Example 2: Urine collection and concentration and subject (patient/control) data

### Urine Collection & storage

Urine from a UK cohort of diabetic patients with and without CKD was analysed for K and N-Cadherin; local ethics committee approval was obtained.

Healthy non-diabetic controls, age range of 20-50, were asked to complete the "Diabetes Risk Score" (Lindstrom & Tuomilehto Diabetes Care 2003); only subjects scoring less than 9 were asked to supply a urine sample. Subjects scoring more than 9 were advised of the relevance and advised to contact their general practitioner.

Approximately 30 mls of urine was collected in a sterile universal container, divided immediately into 2 mls aliquots and stored at -80°C until further analysis. Urine from 24 diabetic patients with eGFR ≥60 mls/min (CKD stages < 3 UK Renal Association guideline), 12 diabetic patients with eGFR<60mls/min (CKD stages ≥3 UK Renal Association guideline) and 12 healthy controls was analysed. The eGFR levels were calculated at the time of urine collection. Anonymised demographic data along with renal functions (creatinine and urine PCR) and HbAlC was tabulated (see Table 1 below). 3-years follow up data was collected for these subjects to assess progression of CKD. Progressors were defined according to the Renal Association guideline; i.e. those in which eGFR as calculated by the MDRD method (abbreviated Modification of Diet in Renal Disease equation: 186 x (Creat / 88.4)^{-1.114} x (Age)^{-0.203} x (0.742 if female) x (1.210 if black)) dropped more than 2mls/min/6months or 5mls/min/year.

**Table 1: Demographic & clinical data for the subject groups studied.**

| **mean** | **eGFR≥60ml/min mean ± SD** | **eGFR<60ml/min mean ± SD** | **Control** |
|---|---|---|---|
| **Age Range (Y)** | 32-80 | 31-80 | 25-60 |
| **Male: Female** | 13:11 | 7:5 | 7:3 |
| **Diabetes Type 2 : Type 1** | 21:3 | 9:3 | None |
| **HbAlC** | 8.44 (1.75) | 8.02 (1.43) | N/A |
| **Mean (STD)** | | | |
| **Urine ACR (mg/mmol)** | 5.1 (9.88) | 109.33 (177.98) | <2.5 |

Table 1: The clinical and demographic data for subjects whose urine was analysed for K and N-cadherin. The clinical characteristics among various groups; diabetic patients without CKD, diabetics with CKD stage <3 and diabetic with CKD stage ≥3 is indicated in tabulated form. The clinical characteristics are shown as mean ± SD among various groups; diabetic patients with CKD stage <3 (eGFR≥60 ml/min) and diabetic patients with CKD stage ≥3 (eGFR <60 ml/min). The non-diabetic healthy controls were volunteers with an age range of 20-50. Diabetes control (blood sugar level control) was fair and not different between diabetic patients with CKD stage <3 and diabetic patients with CKD stage ≥3. As one might predict, diabetic patients with CKD stage ≥3 had higher urinary albumin, RBP and NAG.

### Concentrating urine samples

An aliquot of urine was defrosted, briefly vortexed and spun at 10,000 rpm to remove any particulate matter. 1.5 ml of spun urine supernatant was then transferred to an Amicon® Ultra-4 10K centrifugal filter (10,000 Nominal Molecular Weight Limit, Millipore Corporation, USA) and centrifuged at 3000g for 20-25 min to concentrate the sample 6 fold.

### Example 3: Detection of urinary K and N-cadherin by Western blotting

Urinary K-cadherin was detected by Western Immunoblotting. The samples were prepared for gel electrophoresis by taking 65µl of concentrated urine and adding 25µl of Western sample buffer(x4) and 10µl of reducing agent(x10) (Invitrogen; NP0008, NP0004) and denaturing at 70°C for 10 min. 40µl of samples prepared were loaded on to a 4-12% Bis-Tris gel for electrophoresis followed by immunoblotting. The blot was incubated with mouse monoclonal antibodies against extracellular domain of K-cadherin (diluted 1:1000 in Tris-buffered saline with 0.1% triton and 5% Bovine Serum Albumin, Fischer Thermo-scientific Ltd, UK, Cadherin K Antibody 2B6) and N-cadherin (1:1000, Santa Cruz Biotechnologies, USA). The protein was detected using a rabbit polyclonal anti-mouse IgG (Whole molecule) HRP labelled antibody (Sigma-Aldrich, UK) and Amersham ECL Plus™ Western Blotting Detection Reagents (GE Healthcare Life Sciences, UK) according to the manufacturer's recommendations. The quantification of the band was performed by ImageQuant (GE Healthcare Life Sciences, UK) .

### Results

Western Immunoblot analysis of urinary full length K and N-cadherin.

An example Western blot probed with an antibody to the extracellular domain of K-cadherin is shown in Figure 6, with primary human proximal tubule whole cell lysate as a positive control. The whole molecule of K-cadherin is detectable (∼120 kDa) in the urine from diabetic patients (as judged by gel migration with respect to known standards), with an increasing rate of detection correlating with severity of CKD stages (Figure 6). None of the non-diabetic healthy controls or diabetics with no CKD (data not shown) had detectable K-cadherin in the urine. 75% (9 out of 12) of diabetic subjects with CKD stages ≥3 had detectable K-cadherin in the urine as compared to 29% (7 out of 24) of subjects with CKD<3 and this difference was statistically different (Figure 8).

Western blotting for N-cadherin detected full length protein (∼140 kDa) in the urine from all subjects, patients with diabetes and controls (Figure 7). This data was not analysed further.

This study, using a separate cohort of patients with respect to Example 1, demonstrates that K-cadherin can be found in the urine of diabetic patients with chronic kidney disease, consistent with the loss of expression in the proximal tubule described in Example 1. K-cadherin was not detectable in the urine of any of the healthy controls.

It was hypothesised that cellular degradation and death might lead to cleavage of the K-cadherin molecule and excretion of only the extra-cellular domain in the urine, the cytoplasmic domain being endocytosed and recycled or degraded in the proteasome. Surprisingly, however, full length K-cadherin (as judged by electrophoresis migration) was found in the urine of patients with diabetic CKD, implying release of intact full length K-cadherin from proximal tubular epithelial cells exposed to fibrotic stimuli. Moreover, full length N-cadherin protein was present in urine of all subjects and did not correlate with CKD, which contrasts with the N-cadherin down-regulation in kidney biopsies from patients with diabetic CKD shown in Example 1. This might relate to its wide-spread expression, particularly in the lower urinary tract and in the prostate.

### Example 4: Association of urinary K-cadherin with progression of CKD.

Patients were followed up for up to three years to assess disease progression; one subject was lost from the study during this period.

The level of urine albumin was also assessed for all subjects at the baseline and subsequent time points.

### Statistics

Values were expressed as means ± SD. Diabetic patients with and without various stages of CKD were further stratified into groups based on Progressors and non-Progressors (change of eGFR during 3 years of follow up) and presence or absence of detectable K-cadherin and albumin in the urine at baseline. The difference between groups was evaluated using Chi-Square test in a 2x2 contingency table. All tests were two tailed and p<0.05 was considered significant. Kaplan-Meier event free survival curves were plotted with progression of CKD as an event and presence or absence of K-cadherin or albumin in the urine using Graphpad Prism software. Comparison of these curves was done using log-rank (Mantel-Cox) test. p<0.05 was considered significant. Hazard ratio was calculated at 95% confidence interval. Sensitivity and specificity of urinary K-cadherin and albumin in predicting progression of kidney disease were calculated using standard statistical equations.

### Results

### Diabetics with CKD <3 (eGFR ≥60 ml/min)

Approximately 17% (4 out of 24) diabetic subjects with CKD<3 had progression of their CKD over the 3 year follow up period. All 4 patients had detectable K-cadherin in the urine at the start of the study while only 2 out of these 4 patients had albuminuria (ACR >3). Of those who had stable renal function over this period, 15% (3 out of 20) subjects had detectable K-cadherin in the urine and albuminuria.

Presence or absence of K-cadherin or albumin in the urine of these patients was plotted using Kaplan-Meier survival curve where an event was defined as a progression of CKD (Figure 9). Patients with K-cadherin in the urine had a statistically significant risk of progression of CKD over this follow up period (p<0.0001, HR-75.18 (95% CI of ratio 9.289 to 608.5)), similar to patients with albumin in the urine over this follow up period (p<0.05, HR 16.36 (95% CI of ratio 1.675 to 159.9)) (Figure 9). (HR stands for hazard ratio, CI stands for confidence interval.)

None of the subjects who were negative for urinary K-cadherin at baseline in this group progressed over this follow up period.

In this pilot study, the sensitivity and specificity of urinary K-cadherin for predicting progression of CKD in the CKD <3 group was 100% and 85% respectively whereas that of albuminuria was 50% and 85%. (Sensitivity relates to the ratio of patients who had K-cadherin in urine and progressed to all patients that progressed; specificity relates to the ratio of patients who did not have K-cadherin and did not progress to all patients who did not progress. These are usually expressed as percentages.)

### Diabetics with CKD ≥3 (eGFR <60 ml/min)

41.7% subjects (5 out of 12) from this group had progression of their CKD. All 5 subjects had detectable urinary K-cadherin and albuminuria at baseline. In those with stable CKD (58.3%, 7 out of 12), 42.85% (3 out of 7) and 71.43% (5 out of 7) subjects had detectable urinary K-cadherin and albuminuria respectively (Figure 8).

Presence or absence of K-cadherin or albumin in the urine of these patients was plotted using Kaplan-Meier survival curve where an event was defined as a progression of CKD (Figure 9). Neither patients with K-cadherin in the urine nor patients with albumin in the urine had a statistically significant risk of progression of CKD over this follow up period [(p=0.1047, HR 6.19 95% (95% CI of ratio 0.6846 to 55.97)) and (p=0.1567, HR 4.542 (95% CI of ratio 0.5594 to 36.87)) respectively] (Figure 9). This may be due to small sample size in this group.

None of the subjects who were negative for urinary K-cadherin at the start of the study in this group progressed over this follow up period.

In this pilot study, the sensitivity and specificity of urinary K-cadherin for predicting progression of CKD in the CKD ≥3 group was 100% and 57% respectively, against that of albuminuria which was 100% and 29%.

This Example shows that the presence of urinary K-cadherin correlates with the stage of chronic kidney disease and an increased proportion of patients with detectable urinary k-cadherin had progression of disease over follow-up. Moreover, the prevalence of K-cadherin has a better predictive value than does albuminuria for progression of chronic kidney disease in patients with 'early' diabetic CKD. A similar trend was also noted for patients with advanced CKD, however it is believed that the small sample size prevented this from being found to be statistically significant. Accordingly, K-cadherin is considered to have great value as a marker for separating kidney disease subjects at risk of progression from non-progressors.

Furthermore, the consideration that within the study it can definitely be said that type II diabetes subjects, at both 'early' and 'advanced' disease stages, progressed and had urinary K-cadherin as a marker at baseline, suggests that it may be particularly applicable within the type II diabetic patient population.

### Example 5: ELISA assessment of K-cadherin and association with CKD progression.

An ELISA system as indicated in Figure 12 was devised using commercial reagents. Accordingly, a sandwich ELISA with a capture antibody and a detection antibody, one that targets the near C-terminus and one that targets the N-terminus was established so as to detect the full length molecule. In this example the C-terminal antibody was adhered to the plate wells and used as the capture antibody. The C-terminal antibody was ab64917 purchased from Abcam and the N-terminal antibody was MA1-06305 purchased from Thermo Scientific. A standard curve of K-Cadherin concentration as in Figure 13 was obtained for the ELISA system.

The ELISA protocol is indicated below:

### Antibodies:

Abcam Anti K-cadherin antibody (ab64917), Rabbit polyclonal Thermo Scientific Anti K-cadherin monoclonal antibody (MA1-06305), mouse
Sigma Anti mouse

### Buffers:

Coating buffer - 500mls deionised water add 445mg NA₂CO₃, 1035mg NaHCO₃
Assay buffer - Natural PBS with 0.1% BSA
Wash buffer - Natural PBS with 0.05% Tween 20

### Additional solutions:

Substrate reagent kit RnD (mix equal parts of A and B when needed)
Sulphuric acid (0.125M)
1. Coat plate with Aabcam Anti-K-cadherin. Dilute 1:2000 in coating buffer. 100ul/well. Incubate at 4°C overnight.
2. Make standard curve. Label 7 tubes S1-S7, Label 1 tube Blank.

| | |
|---|---|
| S1 (10,000 ng/ml) | 70ul of K-cadherin stock (100ug/ml) in to 630ul of assay buffer |
| S2 (5000 ng/ml) | 350ul of S1 in to 350ul of assay buffer |
| S3 (2500 ng/ml) | 350ul of S2 in to 350ul of assay buffer. |
| S4 (1250 ng/ml) | 350ul of S3 in to 350ul of assay buffer |
| S5 (625 ng/ml) | 350ul of S4 in to 350ul of assay buffer. |
| S6 (3125ng/ml) | 350ul of S5 in to 350ul of assay buffer. |
| S7 (156 ng/ml) | 350ul of S6 in to 350ul of assay buffer. |
| S8 (78 ng/ml) | 350ul of S7 in to 350ul of assay buffer. |
| S9 (39 ng/ml) | 350ul of S8 in to 350ul of |
| | assay buffer. |
| S10 (19.5 ng/ml) | 350ul of S9 in to 350ul of assay buffer. |

Use assay buffer alone as a blank
3. Wash plate 3 times. 200ul/well. Blot upside down on paper towel to remove excess wash buffer.
4. Add 100ul of standard or samples in duplicate. Incubate on plate shaker for 1 hour at room temperature.
5. Repeat wash, step 2.
6. Add Thermo scientific anti-K-cadherin antibody (mouse monoclonal). Dilute 1:2000 in assay buffer. 100ul/well. Incubate on plate shaker for 1 hour at room temperature.
7. Repeat wash, step 2.
8. Anti-mouse secondary antibody. Dilute 1:40,000 in assay buffer. 100ul/well. Incubate on plate shaker for 1 hour at room temperature.
9. Repeat wash, step 2.
10. Substrate - mix equal parts of A and B. Add 50ul/well of substrate reagent to each well. Incubate for 30-40mins.
11. Stop reaction with 50ul/well of sulphuric acid.
12. Read plate on tecan at 450nm, ref 650nm

Using the ELISA, archived samples (collected with patients' consent to investigate novel biomarkers) from 322 diabetic CKD patients with varying degrees of renal function were analysed. The samples were concentrated 6 fold using PD mini-trap G10 columns from GE Healthcare, then diluted 6 fold in assay buffer to mitigate assay interference by urine matrix components such as organic compounds, pH and electrolytes. In order to identify progressors, national guidelines of a loss in GFR of 5ml/min over 1 year or a loss of GFR of 10ml/min in 5 years were followed.

Of the 322 subjects, 172 were classified as initially having minor renal impairment and 150 as having moderate/severe renal impairment. 49% of patients with mild renal disease exhibited progression for some or all of the 3 to 5 year follow-up period and 34% of patients with moderate/severe disease exhibited progression. The majority of non-progressors in the mild renal impairment group had K-Cad values below the level of detection, a much smaller number of progressors also had values below the level of detection; all such values were classified as 0 K-Cadherin.

In patients with moderate/severe disease, progressors had a mean level of 29.9 ng/ml urinary K-Cadherin while non-progressors had 15.5 ng/ml (p<0.01). Further, in patients with mild disease non-progressors had a mean urinary K-Cadherin of 6.3 ng/ml and the mean concentration in progressors was 12.2 ng/ml (p<0.05).

Though K-cadherin was not detected in all samples, these results indicate that the ELISA is more sensitive than the Western blotting protocols described in previous examples and elevated K-cadherin is strongly associated with disease progression. Furthermore, it seems to be the case that, as measured by the ELISA, urinary concentrations in progressors are, on average, approximately double those of non-progressors for corresponding disease severity states.

## Claims

1. An in vitro method for determining the propensity for a person's diabetic CKD (chronic kidney disease) level to progress in severity, the method comprising testing for the presence/level of K-cadherin in the person's urine to determine if urinary K-cadherin is elevated.

2. A method according to claim 1 wherein the person is a type II diabetic.

3. A method according to claim 1 or 2 wherein elevated K-cadherin is determined with respect to a non-progressive standard which may optionally be for a specific CKD stage, which stage may optionally be mild disease (stages 1/2).

4. A method according to any preceding claim comprising using a K-cadherin cleavage-point specific antibody.

5. A method according to any preceding claim comprising using western immunoblotting or an ELISA (enzyme-linked immunosorbent assay) system for testing for K-cadherin.

6. A method according to claim 5 wherein the ELISA system comprises two sets of antibody samples, one immobilised for capturing K-cadherin and the other for subsequently detecting the protein.

7. A method according to claim 6 wherein one antibody sample is reactive towards the N-terminal end of K-cadherin and the other is reactive towards the C-terminal end.

8. A method according to any preceding claim wherein a urine sample is concentrated before the presence/level of K-cadherin is assessed.

9. Use of a point of care test or kit in a method according to any preceding claim, the test or kit comprising a K-cadherin cleavage-point specific antibody or two sets of K-cadherin antibodies.

10. Use according to claim 9 wherein the test or kit comprises two sets of antibodies and wherein one of the antibody sets is reactive towards the N-terminal end of K-cadherin and the other is reactive towards the C-terminal end.

11. Use according to any of claims 9 to 10 wherein the kit is a kit for an immuno-precipitation assay, such as an ELISA assay.

12. Use of urinary K-cadherin in an in vitro method for determining the propensity for a person's diabetic CKD (chronic kidney disease) level to progress in severity.

## Patentansprüche

1. Ein In-vitro-Verfahren zum Bestimmen der Neigung eines Niveaus einer diabetischen CNK (chronische Nierenkrankheit) einer Person, in einem Schweregrad fortzuschreiten, wobei das Verfahren ein Testen auf das Vorhandensein/Niveau von K-Cadherin in dem Urin der Person umfasst, um zu bestimmen, ob das Urin-K-Cadherin erhöht ist.

2. Ein Verfahren nach Anspruch 1, wobei die Person ein Typ-II-Diabetiker ist.

3. Ein Verfahren nach Anspruch 1 oder 2, wobei erhöhtes K-Cadherin in Bezug auf einen nicht-progressiven Standard bestimmt wird, der optional für ein spezifisches CKD-Stadium gelten kann, wobei das Stadium optional eine leichte Krankheit (Stadien 1/2) sein kann.

4. Ein Verfahren nach einem der vorhergehenden Ansprüche, umfassend ein Verwenden eines für die K-Cadherin-Spaltstelle spezifischen Antikörpers.

5. Ein Verfahren nach einem der vorhergehenden Ansprüche, umfassend das Verwenden von Western-Immunblotting oder einem ELISA(Enzymimmunoassay)-System zum Testen auf K-Cadherin.

6. Ein Verfahren nach Anspruch 5, wobei das ELISA-System zwei Sätze von Antikörperproben umfasst, von denen einer zum Einfangen von K-Cadherin und der andere zum anschließenden Nachweisen des Proteins immobilisiert ist.

7. Ein Verfahren nach Anspruch 6, wobei eine Antikörperprobe gegenüber dem N-terminalen Ende von K-Cadherin reaktiv ist und die andere gegenüber dem C-terminalen Ende reaktiv ist.

8. Ein Verfahren nach einem der vorhergehenden Ansprüche, wobei eine Urinprobe konzentriert wird, bevor das Vorhandensein/Niveau von K-Cadherin bewertet wird.

9. Verwendung eines Schnelltests oder -Kits in einem Verfahren nach einem der vorhergehenden Ansprüche, wobei der Test oder das Kit einen für die K-Cadherin-Spaltstelle spezifischen Antikörper oder zwei Sätze von K-Cadherin-Antikörpern umfasst.

10. Verwendung nach Anspruch 9, wobei der Test oder das Kit zwei Sätze von Antikörpern umfasst und wobei einer der Antikörpersätze gegenüber dem N-terminalen Ende von K-Cadherin reaktiv ist und der andere gegenüber dem C-terminalen Ende reaktiv ist.

11. Verwendung nach einem der Ansprüche 9 bis 10, wobei das Kit ein Kit für einen Immunpräzipitationsassay, wie etwa einen ELISA-Assay, ist.

12. Verwendung des Urin-K-Cadherins in einem In-vitro-Verfahren zum Bestimmen der Neigung des Niveaus der diabetischen CNK (chronische Nierenkrankheit) einer Person, in dem Schweregrad fortzuschreiten.

## Revendications

1. Procédé *in vitro* pour déterminer la propension du niveau de CKD (maladie rénale chronique) diabétique d'une personne à progresser en gravité, le procédé comprenant le test de la présence/du niveau de K-cadhérine dans l'urine de la personne pour déterminer si la K-cadhérine urinaire est élevée.

2. Procédé selon la revendication 1, dans lequel la personne est un diabétique de type II.

3. Procédé selon la revendication 1 ou 2, dans lequel de la K-cadhérine élevée est déterminée par rapport à une norme non progressive qui peut facultativement être pour un stade CKD spécifique, lequel stade peut facultativement être une maladie bénigne (stades 1/2).

4. Procédé selon l'une quelconque des revendications précédentes, comprenant l'utilisation d'un anticorps spécifique à un point de clivage de K-cadhérine.

5. Procédé selon l'une quelconque des revendications précédentes, comprenant l'utilisation d'un immunobuvardage de type Western ou d'un système ELISA (test d'immuno-absorption enzymatique) pour tester la K-cadhérine.

6. Procédé selon la revendication 5, dans lequel le système ELISA comprend deux ensembles d'échantillons d'anticorps, l'un immobilisé pour capturer la K-cadhérine et l'autre pour détecter ultérieurement la protéine.

7. Procédé selon la revendication 6, dans lequel un échantillon d'anticorps est réactif vis-à-vis de l'extrémité N-terminale de la K-cadhérine et l'autre est réactif vis-à-vis de l'extrémité C-terminale.

8. Procédé selon l'une quelconque des revendications précédentes, dans lequel un échantillon d'urine est concentré avant que la présence/le niveau de K-cadhérine soit évalué(e).

9. Utilisation d'un test ou d'un kit de point de soin dans une procédé selon l'une quelconque des revendications précédentes, le test ou le kit comprenant un anticorps spécifique à un point de clivage de K-cadhérine ou deux ensembles d'anticorps de K-cadhérine.

10. Utilisation selon la revendication 9, dans laquelle le test ou le kit comprend deux ensembles d'anticorps et dans lequel l'un des ensembles d'anticorps est réactif vis-à-vis de l'extrémité N-terminale de K-cadhérine et l'autre est réactif vis-à-vis de l'extrémité C-terminale.

11. Utilisation selon l'une quelconque des revendications 9 à 10, dans laquelle le kit est un kit pour un test d'immuno-précipitation, tel qu'un test ELISA.

12. Utilisation de K-cadhérine urinaire dans une procédé *in vitro* pour déterminer la propension du niveau de CKD (maladie rénale chronique) diabétique d'une personne à progresser en termes de sévérité.
